# EUROPÄISCHE PATENTANMELDUNG

(11) **EP 2 474 312 A1**
(43) Veröffentlichungstag der Anmeldung: **11.07.2012**
(21) Anmeldenummer: 12150066.4
(22) Anmeldetag: 03.01.2012
(51) Int. Cl.: A61K 31/4166, A61K 33/04, A61K 35/04, A61P 17/02, A61P 17/04, A61P 17/06, A61K 9/06

(54) **Mittel zur topischen Behandlung von Hauterkrankungen**

(30) Priorität: 06.01.2011 DE 102011000048
(71) Anmelder: Lipp Pharma Arzneimittel GmbH, 59510 Lippetal (DE); Arndt, Hans-Jürgen, 37194 Bodenfelde (DE)
(72) Erfinder: Arndt, Hans-Jürgen, 37194 Bodenfelde (DE)
(74) Vertreter: Christophersen & Partner

(57) **Zusammenfassung**

Mittel zur topischen Behandlung von Hauterkrankungen, enthaltend
A. 0,1 bis 90 Gew.-% Steinkohlenteerlösung,
B. 0,1 bis 40 Gew.-% feinteiligen Schwefel sowie
C. ggf. einen flüssigen oder festen pharmazeutisch annehmbaren Träger oder Verdünnungsstoff, das dadurch gekennzeichnet ist, dass es als weitere Komponente D einen oder mehrere Hydantoine und/oder Hydantoin-Derivate enthält, wobei sich die Gesamtsumme der einzelnen Komponenten jeweils zu 100 Gew.-% addiert.

## Beschreibung

Die Erfindung betrifft ein Mittel zur topischen Behandlung von Hauterkrankungen, insbesondere von entzündlichen und hyperproliferativen Hauterkrankungen bzw. kutanen Manifestationen immunologisch bedingter Erkrankungen, sowie die Verwendung dieser Mittel zur topischen Behandlung von Hauterkrankungen.

Zu den chronisch entzündlichen Hauterkrankungen zählen die Atopische Dermatitis und die Psoriasis vulgaris. Die atopische Dermatitis, auch Neurodermitis oder endogenes Ekzem genannt, hat in den letzten Jahrzehnten einen dramatischen Inzidenzanstieg erfahren, sodass inzwischen nahezu 7 bis 15 % aller Kinder und etwa 3 % der Erwachsenen in der westlichen Welt von dieser Erkrankung betroffen sind. An der Psoriasis vulgaris leiden rund 3 % der Menschen in Mitteleuropa. Die eigentlichen Ursachen dieser Erkrankungen sind bis heute nicht endgültig geklärt, wenngleich es zu einem rasanten Zugewinn an Daten über die Epidemiologie, die Pathophysiologie und den genetischen Hintergrund gekommen ist. Gesichert ist, dass die Disposition zu diesen beiden chronischen Hauterkrankungen vererbt wird. Das therapeutische Spektrum erstreckt sich von der Lokalbehandlung über systemische Therapien bis hin zu alternativen Behandlungsmethoden. Zu den Lokalbehandlungen zählen insbesondere Kortikoide, Vitamin-D-Präparate, Teerzubereitungen, ultraviolette Strahlen (UVB), Photochemotherapie (PUVA) und topische Immunmodulatoren (TIM). Auch systemische Behandlungen werden durchgeführt, beispielsweise Retinoide Zytostatika (z. B. Methotrexat), Immunsuppresiva (z. B. Cyclosporin A), Kortikoide. Zu den alternativen Behandlungsformen zählen Entspannungstechniken, Homöopathie, Gesprächs- und Psychotherapie, Klimabehandlungen und Badekuren. Diese unterschiedlichen Therapieansätze sind Ausdruck dafür, dass eine Heilung bis heute nicht möglich ist. Sie führen oft zu einer substantiellen Verbesserung der Erkrankungen, wobei sie aber auch zum Teil mit erheblichen Nebenwirkungen verbunden sind.

Die bekannten Mittel schaffen in der Regel nur eine kurzfristige Linderung, eine langfristige Linderung oder sogar Heilung dieser Erkrankungen ist bis jetzt nicht möglich.

Aus dem Stand der Technik sind Zusammensetzungen bekannt, die Steinkohlenteerlösung, als weiteren Bestandteil Talkum, Bismutgallat und/oder feinteiligem Schwefel sowie eine flüssigen oder festen pharmazeutisch annehmbaren Träger oder Verdünner enthalten. Diese Zusammensetzung hat jedoch den Nachteil, dass sich die Komponenten nur schwer in Lösung bringen lassen bzw. eine aus diesen Substanzen hergestellte Suspension oder Emulsion nur eine begrenzte Stabilität aufweist. Insbesondere Steinkohlenteer-haltige Zubereitungen haben den Nachteil, dass der Steinkohlenteer empfindliche Inhaltsstoffe zersetzt.

Steinkohlenteer zählt zu den ältesten Wirkstoffen, die bei lokalen Behandlungen auf der Haut zur Therapie chronischer Dermatosen eingesetzt wurden. Die antiproliferative, entzündungshemmende und juckreizstillende Wirkung wird in der wissenschaftlichen Literatur ausführlich beschrieben. Lange Zeit war man der Ansicht, dass Steinkohlenteer eine kanzerogene Wirkung hat, da im Tierversuch bei Steinkohlenteer das Hautkrebsrisiko als erhöht festgestellt wurde. Allerdings konnte auch nachgewiesen werden, dass diese Nebenwirkungen auf den Benzopyren-Gehalt zurückzuführen ist, Benzopyren kommt insbesondere in ungereinigten Steinkohlenteerlösungen vor. Aufgrund dieser neuen Erkenntnisse hat die US-amerikanische Zulassungsbehörde für Arzneimittel (FDA) im Februar 2001 Steinkohlenteer als wirksam und unbedenklich für die Anwendung auf der menschlichen Haut erklärt.

Aufgabe der vorliegenden Erfindung ist es, ein Mittel zur topischen Behandlung von Hauterkrankungen, insbesondere von Psoriasis und atopischer Dermatitis, zur Verfügung zu stellen, das neben den positiven Eigenschaften erkrankter Haut in eine solche galenische Form überführt werden kann, dass das Mittel über einen langen Zeitraum eine konstante Konsistenz aufweist, ohne die Wirksamkeit des Mittels zu beeinträchtigen. Auch sollte unter Abwägung von Risiko und Nutzen einer Lokalbehandlung ein für den Patienten optimales Ergebnis erzielt werden.

Gegenstand der vorliegenden Erfindung ist demgemäß ein Mittel zur topischen Behandlung von Hauterkrankungen, enthaltend
A. 0,1 bis 90 Gew.-% Steinkohlenteerlösung,
B. 0,1 bis 40 Gew.-% feinteiligen Schwefel sowie
C. ggf. einen flüssigen oder festen pharmazeutisch annehmbaren Träger oder Verdünnungsstoff,
   dadurch gekennzeichnet, dass das Mittel als weitere Komponente D ein Hydantoin und/oder Hydantoin-Derivat enthält, wobei sich die Gesamtsumme der einzelnen Komponenten jeweils zu 100 Gew.-% addiert.

Erfindungsgemäß enthält das Mittel als Komponente D ein Hydantoin. Hydantoine sind Produkte des Eiweißstoffwechsels bei Säugetieren und werden üblicherweise mit dem Harn ausgeschieden. In Pflanzen, beispielsweise in der Rosskastanienrinde, Ahorn, Weizenkeim und Beinwell, kommt es ebenfalls vor. Hydantoine haben eine weichmachende Wirkung auf die Hornschicht und beseitigen die Schuppenbildung. Ferner glätten sie die Hautoberfläche, auch wird ihnen eine anti-irritative Wirkung zugeschrieben. Hydantoine sind leicht aus Aldehyden, Ketonen, Aminosäuren und Harnstoffen herstellbar. Besonders bevorzugt werden 5-substituierte Hydantoine eingesetzt, beispielsweise 5-Ureido-Hydantoin. Diese Komponente ist vorzugsweise in einer Menge von 0,01 bis 0,5 Gew.-%, bezogen auf das fertige Mittel, enthalten.

Als Steinkohlenteerlösung der Komponente A kann eine auf dem pharmazeutischen Gebiet handelsübliche Steinkohlenteerlösung eingesetzt werden, die ggf. durch Stabilisatoren, wie Dispergiermittel etc., stabilisiert sein kann, beispielsweise solche, wie sie dem Fachmann auf dem Gebiet der Pharmazie unter der Bezeichnung liqu. carb. deterg. oder liqu. lithan. aceton bekannt sind. Die Menge der Steinkohlenteerlösung ist von der Schwere der Erkrankung und der zu behandelnden Haustelle abhängig. Enthält die eingesetzte Steinkohlenteerlösung große Mengen Lösungsmittel, wie z.B. liqu. lithan. aceton, kann die Komponente A in größeren Mengen eingesetzt werden. Wird das konzentrierte liqu. carb. deterg. eingesetzt, ist die Komponente A im erfindungsgemäßen Mittel vorzugsweise in einer Menge von 0,1 bis 25 Gew.-%, insbesondere von 1 bis 20 Gew.-%, enthalten.

Als Komponente B ist feinteiliger Schwefel enthalten. Es kann jeder auf dem pharmazeutischen Gebiet handelsübliche Schwefel verwendet werden, der auch unter der Bezeichnung Sulf. praec. bekannt ist.

Die Bestandteile der Komponente B können im erfindungsgemäßen Mittel einzeln in Mengen von 0,1 bis 40 Gew.-%, vorzugsweise von 1 bis 20 Gew.-%, eingesetzt werden.

Als Komponente C kann das erfindungsgemäße Mittel einen flüssigen oder festen pharmazeutisch annehmbaren Träger oder Verdünnungsstoff enthalten. Die Komponente C wird in Abhängigkeit von der geplanten Darreichungsform ausgewählt, beispielsweise aus Lösungsmitteln, lipophilen Substanzen etc.

In einigen Fällen hat es sich als vorteilhaft erwiesen, dem erfindungsgemäßen Mittel als Komponente C lipophile Substanzen, d.h. Fette oder Öle zuzusetzen. Diese Fette oder Öle können pflanzlicher oder tierischer Herkunft oder synthetisch hergestellt sein. Vorzugsweise werden pflanzliche Öle, insbesondere Mandelöl, Erdnussöl, Salben, Sesamöl, Olivenöl, Weizenkeimöl, Maiskeimöl, Sojaöl, Sonnenblumenöl, Kokosöl, Avocadoöl, Palmöl, Distelöl und Kakaobutter eingesetzt.

Weiter geeignete lyophile Substanzen sind Wachse, insbesondere Wachse auf natürlicher Basis, wie Walrat, Wollwachs, Bienenwachs, Carnaubawachs oder auch Zuckerrohrwachs. Vorzugsweise enthält das erfindungsgemäße Mittel, wenn es als Salbe vorliegt, ein Gemisch aus einem bei Raumtemperatur festen Wachs und einem bei Raumtemperatur flüssigen Pflanzenöl, insbesondere ein Gemisch aus Bienenwachs und Erdnussöl.

Die Darreichungsformen des erfindungsgemäßen Mittels können die in der pharmazeutischen Industrie üblichen festen und flüssigen Träger- bzw. Verdünnungstoffe enthalten. Bevorzugt werden Träger- und Verdünnungsstoffe, insbesondere bevorzugt sind Wasser, Ethanol, Aceton oder Gemische der voranstehenden. Beispiele von geeigneten galenischen Formen sind Lösungen, Emulsionen, Suspensionen, Lotionen, Gele, Salben oder Cremes, wobei Salbe und Lösungen bevorzugt sind.

Das Mittel kann als Komponente E noch weitere pharmazeutische Hilfs- und Wirkstoffe enthalten, die die Wirkung der erfindungsgemäß enthaltenen Komponenten unterstützen. Außerdem kann das Mittel für die jeweilige galenische Form übliche Bestandteile einschließlich Stabilisatoren enthalten.

In einer bevorzugten Ausführungsform enthält das Mittel als weitere Komponente E einen Duftstoff und/oder Feuchtigkeit-bindende Mittel.

Als Duftstoffe sind beliebige Duftstoffe geeignet, die in dem Mittel nicht angegriffen oder zersetzt werden und deren Duftwirkung ausreichend ist, um die Eigengerüche der Inhaltsstoffe A und B zu überdecken. Als weitere aromatisierende Komponenten können Essenzen auf der Basis von Gewürz- und Heilkräutern eingesetzt werden, wie auf der Basis von Rosmarin, Lavendel, Thymian, Salbei, Citrusaromen, Zitronenmelisse, Minze und ggf. auch ölhaltigen Gewürzen. Duftstoffe sind vorzugsweise in einer Menge bis zu 2 Gew.-%, vorzugsweise bis zu 1 Gew.-% enthalten.

Zu den Feuchtigkeit-bindenden Substanzen zählt beispielsweise Harnstoff. Feuchtigkeit-bindenden Mittel können in einer Menge von 1 bis 10 Gew.-% enthalten sein.

Das erfindungsgemäße Mittel kann durch in der pharmazeutischen Industrie für die jeweilige galenische Form übliche Verfahren hergestellt werden. Die erfindungsgemäß enthaltenen Substanzen sowie ggf. weitere Komponenten werden gut vermischt, so dass diese gleichmäßig verteilt sind. Zur Stabilisierung des erhaltenen Mittels können ggf. übliche Stabilisatoren zugesetzt werden.

In einer möglichen Ausführungsform liegt das erfindungsgemäße Mittel als Salbe vor. In dieser Ausgestaltung ist es bevorzugt, wenn die Träger und Verdünnungsstoffe der Komponente C eine Salbengrundlage sind. Beispielsweise kann die Salbengrundlage ausgewählt sein aus einem bei Raumtemperatur festen Wachs und einem bei Raumtemperatur flüssigen Pflanzenöl. Beispiele für geeignete Salbengrundlagen sind Unguentum leniens, Unguentum emulsificans, Unguentum emulsificans aquosum, Unguentum cetomacrogolis, Unguentum cetylicum cum aqua, Unguentum Alcoholum Lanae bzw. Unguentum adeps lanae, Unguentum molle und/oder Unguentum Zinci.

Eine bevorzugt eingesetzte Salbengrundlage ist ein Gemisch aus Bienenwachs und Erdnussöl. Im Handel ist dieses Gemisch auch als Unguentum Leniens bekannt, es handelt sich hierbei um ein Gemisch aus gelbem Wachs, Cetylpalmitat, Erdnussöl und Wasser. In einer bevorzguten Ausführungsform wird die Salbengrundlage Unguentum Leniens in einer solchen Menge eingesetzt, dass das erfindungsgemäße Mittel 3,0 bis 10,0 Gew.-% Cera alba (Bienenwachs), 1 bis 16 Gew.-% Cetylpalmitat, 40,0 bis 80,0 Gew.-% Erdnussöl, 15,0 bis 35,0 Gew.-% Wasser enthält.

Die der Salbengrundlage ist vorzugsweise in einer Menge von 40 bis 95 Gew.-%, insbesondere von 40 bis 90 Gew.-% und besonders bevorzugt von 60 bis 90 Gew.-%, bezogen auf die fertige Salbe, enthalten.

In einer bevorzugten Ausführungsform enthält das erfindungsgemäße Mittel
A. 0,1 bis 20 Gew.-% Steinkohlenteerlösung,
B. 0,1 bis 40 Gew.-% feinteiligen Schwefel,
C. 40 bis 95 Gew.-%, insbesondere 60 bis 90 Gew.-% Salbengrundlage
D. 0,01 bis 0,5 Gew.-% Hydantoin oder ein Hydantoin-Derivat,
E. ggf. weitere pharmazeutische Hilfs- und Wirkstoffe,
wobei sich die Gesamtsumme der einzelnen Komponenten jeweils zu 100 % addiert.

In einer besonders bevorzugten Ausführungsform enthält das erfindungsgemäße Mittel
A. 0,1 bis 20 Gew.-% Steinkohlenteerlösung,
B. 0,1 bis 40 Gew.-% feinteiligen Schwefel,
C. 3,0 bis 10,0 Gew.-% Cera alba (Bienenwachs), 1 bis 16 Gew.-% Cetylpalmitat, 40,0 bis 80,0 Gew.-% Erdnussöl, 15,0 bis 35,0 Gew.-% Wasser,
D. 0,01 bis 0,5 Gew.-% Hydantoin oder ein Hydantoin-Derivat,
E. ggf. weitere pharmazeutische Hilfs- und Wirkstoffe
wobei sich die Gesamtsumme der einzelnen Komponenten jeweils zu 100 % addiert.

Die Herstellung des erfindungsgemäßen Mittels kann in an sich bekannter Weise zur Vermischen oder Verrühren der einzelnen Komponenten erfolgen. Besonders stabile Zusammensetzungen werden erhalten, wenn zu nächst die Komponenten A und D, ggf. in Gegenwart eines üblichen Trägers oder Verdünnungsstoffs, vermischt und danach die weiteren Komponenten hinzugefügt werden. Anschließend kann die Zusammensetzung noch durch Zusatz von weiterem Verdünndungsstoff oder Träger auf die gewünschte Menge aufgefüllt werden.

Für die obige Verwendung hängt die zu verabreichende Dosis von der verwendeten Verbindung, der Verabreichungsform sowie der Behandlungsart ab. Es werden sehr gute Ergebnisse erhalten, wenn das Mittel dünn auf die erkrankten Hautstellen aufgetragen wird. In der Regel ist es ausreichend, das Mittel ein- bis zweimal aufzutragen. Sollte nach einem Zeitraum von einigen Tagen bis zu einer Woche noch keine deutliche Linderung eingetreten sein, kann das Mittel nochmals aufgebracht werden.

Durch Anwendung des erfindungsgemäßen Mittels ist es möglich, die Symptome von Hauterkrankungen, insbesondere von chronischen Hauterkrankungen, wie entzündlichen und hyperproliferativen Hauterkrankungen wie Psoriasis, auch atopischer Dermatitis (Neurodermitis), Kontakt-Dermatitis und anderen ekzematösen Dermatitiden, seborrhoeischer Dermatitis, langfristig zu lindern und in vielen Fällen sogar zu heilen. Besonders hilfreich ist das Mittel bei Psoriasis und atopischer Dermatitis (Neurodermitis).

### Beispiele

### Herstellungsbeispiele

### 1. Rezeptur für eine Salbe

| **Inhaltsstoff** | **Menge/Gew.-%** | |
|---|---|---|
| Liquor carbon Detergens | 7,5 | 7.5 |
| feinteiliger Schwefel | 7,5 | 7,5 |
| 5-Ureido-Hydantoin | - | 0,1 |
| Salbengrundlage unguentum leniens | 85,0 | 84,9 |

Über einen Zeitraum von 2 Jahren wurden sechs Patienten mit Psoriasis vulgaris (Plaque-Form) und vier Patienten mit atomischer Dermatitis behandelt. Die Salbe wurde zweimal täglich dünn aufgetragen. Bei allen Patienten klang der Juckreiz innerhalb weniger Tage ab und nach vier bis sechs Wochen waren die erkrankten Bereiche vollständig abgeheilt.

## Patentansprüche

1. Mittel zur topischen Behandlung von Hauterkrankungen, enthaltend
A. 0,1 bis 90 Gew.-% Steinkohlenteerlösung,
B. 0,1 bis 40 Gew.-% feinteiligen Schwefel sowie
C. ggf. einen flüssigen oder festen pharmazeutisch annehmbaren Träger oder Verdünnungsstoff,
**dadurch gekennzeichnet, dass** das Mittel als weitere Komponente D ein Hydantoin und/oder ein Hydantoin-Derivat enthält, wobei sich die Gesamtsumme der einzelnen Komponenten jeweils zu 100 Gew.-% addiert.

2. Mittel nach Anspruch 1, **dadurch gekennzeichnet, dass** die Komponente D 5-Ureido-Hydantoin ist.

3. Mittel nach einem der Ansprüche 1 oder 2, **dadurch gekennzeichnet, dass** die Komponente D in einer Menge von 0,01 bis 0,5 Gew.-% enthalten ist.

4. Mittel nach einem der Ansprüche 1 bis 3, **dadurch gekennzeichnet, dass** es in Form einer Salbe vorliegt.

5. Mittel nach Anspruch 4, **dadurch gekennzeichnet, dass** die Komponente C ausgewählt ist aus einer der Salbengrundlagen Unguentum leniens, Unguentum emulsificans, Unguentum emulsificans aquosum, Unguentum cetomacrogolis, Unguentum cetylicum cum aqua, Unguentum Alcoholum Lanae bzw. Unguentum adeps lanae, Unguentum molle und/oder Unguentum Zinci.

6. Mittel nach Anspruch 4 oder 5, **dadurch gekennzeichnet, dass** die Salbengrundlage Unguentum leniens ist und ein Gemisch aus Bienenwachs, Erdnussöl und Wasser und als Cetylpalmitat enthält.

7. Mittel nach Anspruch 6, **dadurch gekennzeichnet, dass** es 3,0 bis 10,0 Gew.-% Cera alba (Bienenwachs), 1 bis 16 Gew.-% Cetylpalmitat, 40,0 bis 80,0 Gew.-% Erdnussöl, 15,0 bis 35,0 Gew.-% Wasser enthält.

8. Mittel nach einem der Ansprüche 1 bis 7, **dadurch gekennzeichnet, dass** das es als weitere Komponente E einen Duftstoff enthält.

9. Mittel nach einem der Ansprüche 1 bis 8, **dadurch gekennzeichnet** es enthält
A 0,1 bis 20 Gew.-% Steinkohlenteerlösung,
B 0,1 bis 40 Gew.-% feinteiligen Schwefel,
C 40 bis 95 Gew.-%, insbesondere 60 bis 90 Gew.-% Salbengrundlage
D 0,01 bis 0,5 Gew.-% Hydantoin oder ein Hydantoin-Derivat,
E ggf. weitere pharmazeutische Hilfs- und Wirkstoffe,
wobei sich die Gesamtsumme der einzelnen Komponenten jeweils zu 100 % addiert.

10. Mittel nach einem der Ansprüche 1 bis 9, **dadurch gekennzeichnet, dass** es enthält
A 0,1 bis 20 Gew.-% Steinkohlenteerlösung,
B 0,1 bis 40 Gew.-% feinteiligen Schwefel,
C 3,0 bis 10,0 Gew.-% Cera alba (Bienenwachs), 1 bis 16 Gew.-% Cetylpalmitat, 40,0 bis 80,0 Gew.-% Erdnussöl, 15,0 bis 35,0 Gew.-% Wasser,
D 0,01 bis 0,5 Gew.-% Hydantoin oder ein Hydantoin-Derivat,
E ggf. weitere pharmazeutische Hilfs- und Wirkstoffe,
wobei sich die Gesamtsumme der einzelnen Komponenten jeweils zu 100 % addiert.

11. Verwendung eines Mittels nach einem der Ansprüche 1 bis 10 zur topischen Anwendung bei Hauterkrankungen.

12. Verwendung nach Anspruch 11, **dadurch gekennzeichnet, dass** es sich bei den Hauterkrankungen um Psoriasis und atopische Dermatitis, handelt.
